(19) **Europäisches Patentamt / European Patent Office / Office européen des brevets**

(11) **EP 2 052 673 B1**

(12) **EUROPEAN PATENT SPECIFICATION**

(45) Date of publication and mention
of the grant of the patent:
**15.11.2017 Bulletin 2017/46**

(21) Application number: **07742106.3**

(22) Date of filing: **20.04.2007**

(51) Int Cl.:
*A61B 1/04* (2006.01)    *A61B 1/00* (2006.01)
*A61B 1/06* (2006.01)    *G02B 23/24* (2006.01)
*H04N 7/18* (2006.01)

(86) International application number:
**PCT/JP2007/058671**

(87) International publication number:
**WO 2008/020499 (21.02.2008 Gazette 2008/08)**

(54) **ENDOSCOPIC DEVICE AND ITS PROCESSING METHOD**

ENDOSKOPVORRICHTUNG UND AUFBEREITUNGSVERFAHREN

DISPOSITIF ENDOSCOPE ET SON PROCEDE DE TRAITEMENT

(84) Designated Contracting States:
**DE FR GB**

(30) Priority: **18.08.2006 JP 2006223576**

(43) Date of publication of application:
**29.04.2009 Bulletin 2009/18**

(73) Proprietor: **Olympus Corporation
Tokyo 192-8507 (JP)**

(72) Inventors:
• **YAMAZAKI, Kenji
Tokyo 151-0072 (JP)**

• **GONO, Kazuhiro
Tokyo 151-0072 (JP)**

(74) Representative: **von Hellfeld, Axel
Wuesthoff & Wuesthoff
Patentanwälte PartG mbB
Schweigerstrasse 2
81541 München (DE)**

(56) References cited:
**EP-A- 2 047 792      JP-A- 2002 095 635
JP-A- 2003 093 336      JP-A- 2006 061 621
US-A1- 2003 229 270**

**Description**

Technical Field

The present invention relates to an endoscope apparatus and particularly relates to an endoscope apparatus which picks up an image of a living tissue and performs signal processing, and a signal processing method of the apparatus.

Background Art

Conventionally, endoscope apparatuses have been widely used which apply illumination light to obtain endoscope images in body cavities. In such an endoscope apparatus, an electronic endoscope is used which has image pickup means for guiding illumination light into a body cavity from a light source device with a light guide and the like and picking up an image of a subject through return light. By performing signal processing on an image pickup signal from the image pickup means by a video processor, an endoscope image is displayed on an observation monitor to enable observation of an observation part such as a diseased part.

When a normal observation of a living tissue is performed in the endoscope apparatus, white light of a visible light region is emitted by the light source device. Frame sequential light is applied to a subject through, for example, an RGB rotary filter and the like, and return light obtained from the frame sequential light is synchronized and is subjected to image processing by the video processor, so that a color image is obtained. Alternatively, a color chip is placed at a front of an image pickup surface of the image pickup means in the endoscope, and the return light obtained from white light is separated into color components to pick up an image and the image is subjected to image processing by the video processor, so that a color image is obtained.

On a living tissue, light absorption characteristics and scattering characteristics vary with the wavelength of applied light. For example, Japanese Patent Application Laid-Open Publication No. 2002-95635 proposes a narrow-band light endoscope apparatus which emits illumination light of a visible light region, irradiates a living tissue with narrow-band RGB frame sequential light having discrete spectral characteristics, and obtains tissue information at a desired depth of the living tissue.

JP 2006/061621 and US 2003/0229270 A1 disclose endoscope devices for which normal light observation and narrow band light observation are performed separately.

However, in order to observe normal light and narrow-band light in the endoscope apparatus of the prior art, it is necessary to apply the normal light and the narrow-band light to a living tissue at different times, resulting in complicated configurations of a light source and an optical filter.

Since normal light and narrow-band light are applied to the living tissue at different times, the same living tissue cannot be simultaneously observed in real time in a normal light observation image and narrow-band light observation.

The present invention has been devised in view of the above point. An object of the present invention is to provide an endoscope apparatus and a signal processing method of the apparatus which can simultaneously observe a same living tissue in real time in a normal light observation image and narrow-band light observation with a simple configuration.

Disclosure of Invention

Means For Solving the Problem

An endoscope apparatus according to claim 1 and a method according to claim 9 are provided. The endoscope apparatus may include:

biological image information acquiring means for receiving a subject image of the subject having been irradiated with the illumination light from the illuminating means, and obtaining biological image information of the subject;
band limiting means which is disposed on an optical path from the illuminating means to the biological image information acquiring means and limits, to a predetermined bandwidth, at least one of a plurality of wavelength bands allocated according to penetration depths of light in the subject;
biological image information converting means for converting the biological image information obtained by the biological image information acquiring means, to first biological image signal information corresponding to irradiation with band limited light of the plurality of wavelength bands with the predetermined bandwidth and second biological image information corresponding to irradiation with the illumination light; and
display image generating means for generating a display image to be displayed on display means, based on the first biological image signal information and the second biological image signal information which have been converted by the biological image information converting means.

A signal processing method of an endoscope apparatus according to an aspect of the present invention includes:

an illuminating step of applying illumination light to a subject;
a biological image information acquiring step of receiving a subject image of the subject having been irradiated with the illumination light, and obtaining biological image information of the subject;
a band limiting step of limiting, to a predetermined bandwidth, at least one of a plurality of wavelength bands allocated according to penetration depths of light in the subject, on an optical path from the illuminating means to the biological image information acquiring means;
a biological image information converting step of converting the biological image information obtained in the biological image information acquiring step, to first biological image signal information corresponding to irradiation with band limited light of the plurality of wavelength bands with the predetermined bandwidth and second biological image information corresponding to irradiation with the illumination light; and
a display image generating step of generating a display image to be displayed on display means, based on the first biological image signal information and the second biological image signal information which have been converted in the biological image information converting step.

Brief Description of the Drawings

Fig. 1 is a structural diagram showing a configuration of an endoscope apparatus according to a first embodiment of the present invention;
Fig. 2 is a structural diagram showing a configuration of a rotary filter shown in Fig. 1;
Fig. 3 is a diagram showing spectral characteristics of a filter set of the rotary filter;
Fig. 4 is a structural diagram showing a configuration of a respective band signal conversion section shown in Fig. 1;
Fig. 5 is a diagram showing amplitude characteristics of a BPF shown in Fig. 4;
Fig. 6 is a first diagram showing a display example of an observation monitor shown in Fig. 1;
Fig. 7 is a second diagram showing a display example of the observation monitor shown in Fig. 1;
Fig. 8 is a third diagram showing a display example of the observation monitor shown in Fig. 1;
Fig. 9 is a diagram showing $\gamma$ correction characteristics of a $\gamma$ correction circuit shown in Fig. 1;
Fig. 10 is a structural diagram showing a configuration of an endoscope apparatus according to a second embodiment of the present invention;
Fig. 11 is a structural diagram showing a configuration of a primary color filter shown in Fig. 10;
Fig. 12 is a diagram showing a transmission property of the primary color filter shown in Fig. 11;
Fig. 13 is a structural diagram showing a configuration of a respective band signal conversion section shown in Fig. 10;
Fig. 14 is a structural diagram showing a configuration of an endoscope apparatus according to a third embodiment of the present invention;
Fig. 15 is a diagram showing a transmission property of a heat ray cut-off filter shown in Fig. 14;
Fig. 16 is a structural diagram showing a configuration of a complementary color filter shown in Fig. 14;
Fig. 17 is a structural diagram showing a configuration of a respective band signal conversion section shown in Fig. 14;
Fig. 18 is a diagram showing a modification of a transmission property of the heat ray cut-off filter shown in Fig. 14; and
Fig. 19 is a structural diagram showing a configuration of a modification of a respective band signal conversion section shown in Fig. 14.

Best Mode for Carrying Out the Invention

Embodiments of the present invention will be described below in accordance with accompanying drawings.

(First Embodiment)

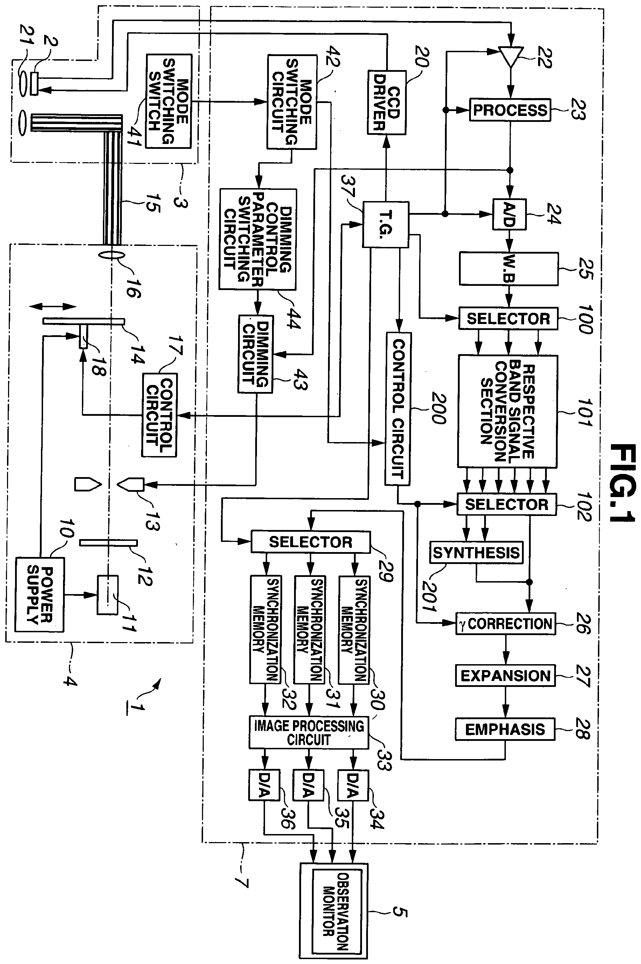

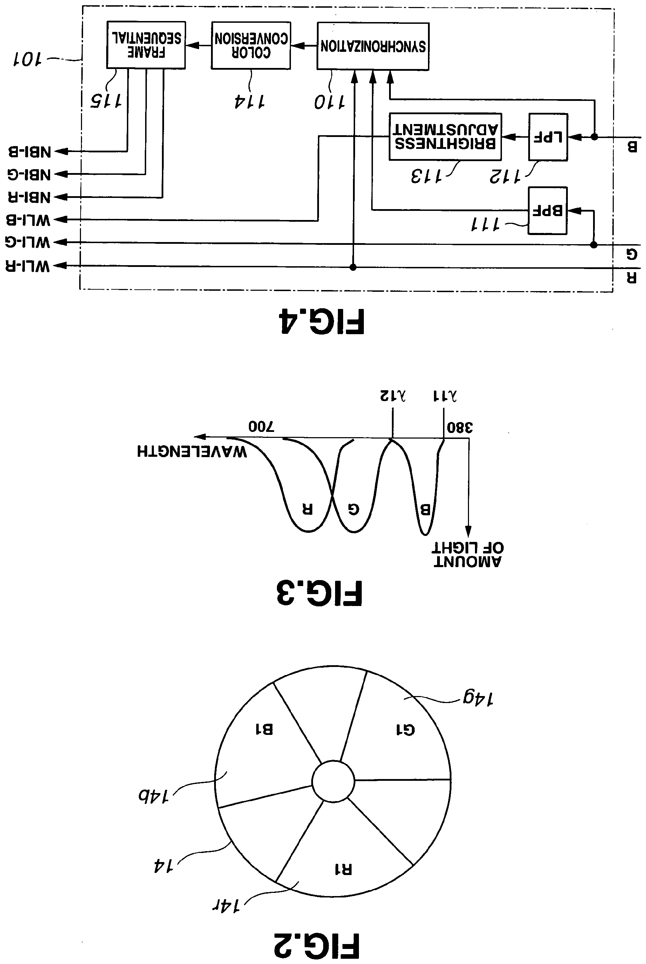

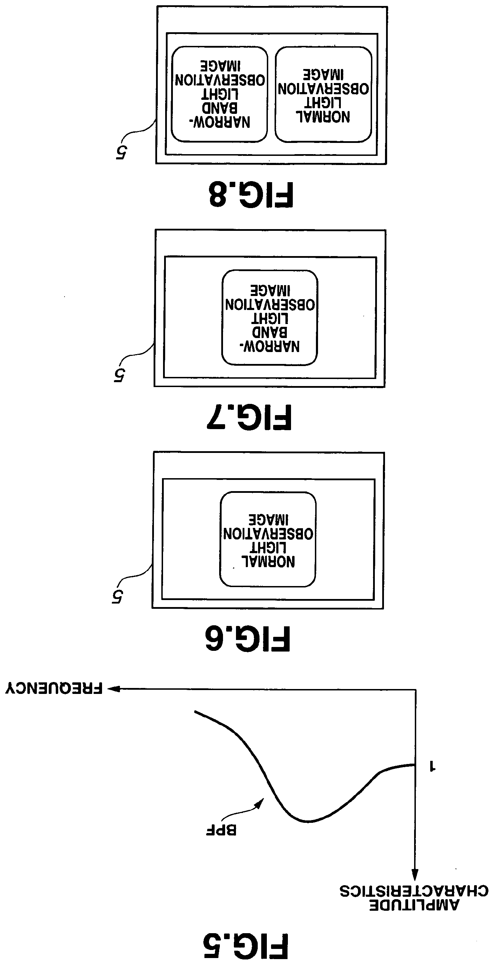

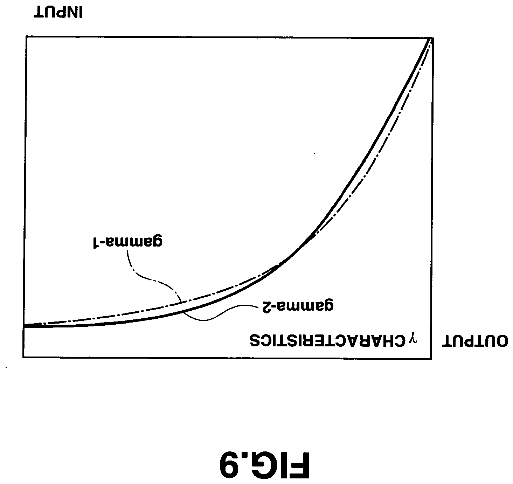

Figs. 1 to 9 show a first embodiment of the present invention. Fig. 1 is a structural diagram showing a configuration of an endoscope apparatus. Fig. 2 is a structural diagram showing a configuration of a rotary filter shown in Fig. 1. Fig. 3 shows spectral characteristics of a filter set of the rotary filter shown in FIG. 2. Fig. 4 is a structural diagram showing a configuration of a respective band signal conversion section shown in Fig. 1. Fig. 5 shows amplitude characteristics of a BPF shown in Fig. 4. Fig. 6 is a first diagram showing a display example of an observation monitor shown in Fig. 1. Fig. 7 is a second diagram showing a display example of the observation monitor shown in Fig. 1. Fig. 8 is a third diagram showing a display example of the observation monitor shown in Fig. 1. Fig. 9 shows γ correction characteristics of a γ correction circuit shown in Fig. 1.

As shown in Fig. 1, an endoscope apparatus 1 of the present embodiment is made up of an electronic endoscope 3 which has a CCD 2 acting as biological image information acquiring means to be inserted into a body cavity to pick up an image of a tissue in the body cavity, a light source device 4 for supplying illumination light to the electronic endoscope 3, and a video processor 7 for performing signal processing on an image pickup signal from the CCD 2 of the electronic endoscope 3 and displaying an endoscope image on an observation monitor 5.

The light source device 4 includes a xenon lamp 11 acting as illuminating means for emitting illumination light (white light), a heat ray cut-off filter 12 for cutting off heat rays of white light, a beam limiting device 13 for controlling an amount of white light having passed through the heat ray cut-off filter 12, a rotary filter 14 acting as band limiting means for limiting illumination light to frame sequential light, a condenser lens 16 for condensing frame sequential light, which has passed through the rotary filter 14, on an incidence plane of a light guide 15 disposed in the electronic endoscope 3, and a control circuit 17 for controlling a rotation of the rotary filter 14.

As shown in Fig. 2, the rotary filter 14 is disc-sliaped and has a rotation axis at a center of the filter. In a radiant part of the rotary filter 14, an R filter portion 14r, a G filter portion 14g, and a B filter portion 14b are disposed which compose the filter set for outputting frame sequential light having spectral characteristics shown in Fig. 3. The R filter portion 14r and the G filter portion 14g have overlapping spectral characteristics, and the spectral characteristics of the B filter portion 14b have a narrow band of 405 nm to 425 nm, for example, in a wave range of λ11 to λ12. The spectral characteristics may have a narrow band of 400 nm to 440 nm in the wave range of λ11 to λ12 of the B filter portion 14b.

As shown in Fig. 1, the rotary filter 14 is rotated by performing drive control on a rotary filter motor 18 through the control circuit 17.

The xenon lamp 11, the beam limiting device 13, and the rotary filter motor 18 are fed with power from a power supply section 10.

The video processor 7 includes a CCD driver 20, an amplifier 22, a process circuit 23, an A/D converter 24, a white balance circuit (W.B) 25, a selector 100, a respective band signal conversion section 101 acting as biological image information converting means, a selector 102, a γ correction circuit 26, an expansion circuit 27, an emphasis circuit 28, a selector 29, synchronization memories 30, 31, and 32, an image processing circuit 33, D/A circuits 34, 35, and 36, a timing generator (T.G) 37, a control circuit 200, and a synthesis circuit 201 acting as display image generating means.

The CCD drive circuit 20 drives the CCD 2 provided in the electronic endoscope 3 and outputs a frame sequential image pickup signal synchronized with a rotation of the rotary filter 14. The amplifier 22 amplifies the frame sequential image pickup signal which has been obtained by picking up an image in a body cavity with the CCD 2 through an objective optical system 21 provided on an end of the electronic endoscope 3.

The process circuit 23 performs correlated dual sampling, noise removal, and the like on the frame sequential image pickup signal having passed through the amplifier 22. The A/D converter 24 converts the frame sequential image pickup signal, which has passed through the process circuit 23, to a digital frame sequential image signal.

The W.B 25 performs gain control and white balance processing on the frame sequential image signal, which has been digitized by the A/D converter 24, such that an R signal of the image signal and a B signal of the image signal have an equal brightness relative to a G signal of the image signal, for example (in other words, the W.B 25 obtains the R, G, and B signals when a subject has a white surface, for example, in a state in which a white cap is attached to the end of the electronic endoscope 3, and the W.B 25 multiplies the R signal and the B signal by a gain coefficient calculated based on a ratio of brightness relative to the G signal, so that white balance processing is performed so as to generate the R and B signals with a brightness equal to the brightness of the G signal).

The selector 100 outputs the frame sequential image signal from the W.B 25 dividedly to parts of the respective band signal conversion section 101. The respective band signal conversion section 101 converts the image signal from the selector 100, to a normal light observation image signal and a narrow-band light observation image signal. The selector 102 sequentially outputs the frame sequential image signals of the normal light observation image signal and the narrow-band light observation image signal from the respective band signal conversion section 101, to the γ correction circuit 26 and the synthesis circuit 201.

The γ correction circuit 26 performs γ correction on the frame sequential image signal from the selector 102 or the synthesis circuit 201. The expansion circuit 27 expands the frame sequential image signal having been subjected to γ correction by the γ correction circuit 26. The emphasis circuit 28 performs edge enhancement on the frame sequential image signal having been expanded by the expansion circuit 27. The selector 29 and the synchronization memories 30, 31, and 32 are provided to synchronize the frame sequential image signal from the emphasis circuit 28.

The image processing circuit 33 reads the frame sequential image signals stored in the synchronization memories 30, 31, and 32, and corrects a moving image color drift and so on. The D/A circuits 34, 35, and 36 convert the image signal from the image processing circuit 33 to analog video signals and output the signals to the observation monitor 5. The T.G 37 is fed with a sync signal, which has been synchronized with a rotation of the rotary filter 14, from the control circuit 17 of the light source device 4 and outputs various timing signals to the circuits in the video processor 7.

The electronic endoscope 2 further includes a mode switching switch 41 for feeding an output to a mode switching circuit 42 in the video processor 7. The mode switching circuit 42 of the video processor 7 outputs a control signal to a dimming control parameter switching circuit 44 and the control circuit 200. A dimming circuit 43 controls the beam limiting device 13 of the light source device 4 based on the dimming control parameter from the dimming control parameter switching circuit 44 and the image pickup signal having passed through the process circuit 23, so that a brightness is properly controlled.

Referring to Fig. 4, the respective band signal conversion section 101 will be described below. The selector 100 sequentially outputs the frame sequential image signals (respective color signals) from the W.B 25 to the respective band signal conversion section 101 based on the timing signals from the T.G 37.

As shown in Fig. 4, in the respective band signal conversion section 101, the R signal which is the color signal from the selector 100 is a wide-band R image signal suitable for a normal observation. The R signal is outputted through the respective band signal conversion section 101 to the selector 102 as a normal light observation R signal (hereinafter, will be referred to as WLI-R), and the R signal is outputted to a synchronization memory 110.

Further, in the respective band signal conversion section 101, the G signal which is the color signal from the selector 100 is a wide-band G image signal suitable for a normal observation. The G signal is passed through the respective band signal conversion section 101 and is outputted to the selector 102 as a normal light observation G signal (hereinafter, will be referred to as WLI-G), and the G signal is outputted to the synchronization memory 110 through a band-pass filter (BPF) 111. Since the G signal is passed through the BPF 111 having the amplitude characteristics of Fig. 5, a contrast is increased in tissue information on a deep portion reproduced by the wide-band G image signal and a high contrast image signal is generated which corresponds to an image obtained by irradiation with illumination light having spectral characteristics with a narrower band than illumination light having passed through the G filter portion 14g.

Moreover, in the respective band signal conversion section 101, the B signal which is the color signal from the selector 100 is outputted to the synchronization memory 110, is subjected to a predetermined brightness adjustment performed in a brightness adjustment circuit 113 through a low-pass filter (LPF) 112, and is outputted to the selector 102 as a normal light observation B signal (hereinafter, will be referred to as WLI-B). The B signal which is the color signal from the selector 100 is a narrow-band B image signal suitable for a narrow-band light observation. Since the B signal is passed through the LPF 112, a low-contrast image is generated which is equivalent to an image obtained by irradiation with illumination light having spectral characteristics with a wider band than illumination light having passed through the B filter portion 14b. Further, the B image signal is an image signal obtained by irradiation with narrow-band light on a blue short-wavelength side. Light is considerably absorbed by blood and so on and thus darkness increases. Therefore, the brightness adjustment circuit 113 is provided in the post-stage of the LPF 112 to adjust a brightness to a desired brightness and output the B signal as WLI-B to the selector 102.

The color signals inputted to the synchronization memory 110 are subjected to predetermined color conversion by a color conversion circuit 114 as expressed in formula (1) and are outputted to the selector 102 through a frame sequential circuit 115 as a frame sequential narrow-band light observation R signal (hereinafter, will be referred to as NBI-R), a frame sequential narrow-band light observation G signal (hereinafter, will be referred to as NBI-G) and a frame sequential narrow-band light observation B signal (hereinafter, will be referred to as NBI-B).

[Formula 1]

$$\begin{pmatrix} NBI - R \\ NBI - G \\ NBI - B \end{pmatrix} = \begin{pmatrix} 0 & m_1 & 0 \\ 0 & 0 & m_2 \\ 0 & 0 & m_3 \end{pmatrix} \begin{pmatrix} r \\ g \\ b \end{pmatrix} \qquad \cdots (1)$$

where m1, m2, and m3 represent color conversion coefficients (real numbers) and r, g, and b represent color signals of R, G, and B which are inputted to the color conversion circuit 114.

The selector 102 outputs the frame sequential color signals of WLI-R, WLI-G, and WLI-B which compose the normal light observation image and the frame sequential color signals of NBI-R, NBI-G, and NBI-B which compose the narrow-band light image to the γ correction circuit 26 or the synthesis circuit 201 based on the control signal from the control circuit 200.

The image processing circuit 33 makes a moving image color drift correction to the color signals inputted from the synchronization memories 30, 31, and 32 and generates image signals to be outputted to the D/A circuits 34, 35, and 36. In other words, when the frame sequential color signals of WLI-R, WLI-G, and WLI-B are inputted, the image processing circuit 33 generates the normal light observation image. When the frame sequential color signals of NBI-R, NBI-G, and NBI-B are inputted, the image processing circuit 33 generates the narrow-band light image. When the image processing circuit 33 is fed with frame sequential color signals of a synthetic image signal which will be described later, the image processing circuit 33 generates a synthetic image signal having been subjected to a moving image color drift correction.

Further, as shown in Figs. 6 and 7, the normal light observation image and the narrow-band light image are displayed on the observation monitor 5 while being switched in real time in a toggling manner in response to an operation of the mode switching switch 41. Moreover, as shown in FIG. 8, the normal light observation image and the narrow-band light image can be displayed in real time on the same screen of the observation monitor 5 in response to an operation of the mode switching switch 41.

In other words, in the present embodiment, the selector 102 is switched based on the control signal from the control circuit 200 to input two image signals of the same color signal (in the case of the R signal, WLI-R and NBI-R) to the synthesis circuit from memories (not shown) included in the selector 102, in a display mode for simultaneously displaying the normal light observation image and the narrow-band light observation image on the observation monitor 5.

The synthesis circuit 201 reduces the two inputted image signals and then synthesizes the image signals, so that a synthetic image signal is generated. The synthesis circuit 201 outputs the generated signal to the $\gamma$ correction circuit 26 (the G and B signals are similarly synthesized, and WLI-R and NBI-R, WLI-G and NBI-G, and WLI-B and NBI-B are controlled based on the control signal from the control circuit 200, which will be described later, such that the signals are sequentially inputted to the synthesis circuit 201, the synthetic image signal being outputted from the synthesis circuit 201 to the $\gamma$ correction circuit 26 in a frame sequential manner).

In a mode for displaying only one of the normal light observation image and the narrow-band light observation image, the selector 102 is not switched to output the image signals to the synthesis circuit 201 based on the control signal from the control circuit 200 but is switched to output the R signal, the G signal, and B signal of the normal light observation image or the narrow-band light observation image to the $\gamma$ correction circuit 26 in a frame sequential manner.

The control circuit 200 identifies the mode based on a mode switching signal from the mode switching circuit 42 and switches the selector 102. After that, the control circuit 200 controls the R, G, and B signals in the selector 102 based on the timing signal from the T.G 37 such that the signals are sequentially outputted to the synthesis circuit 201 or the $\gamma$ correction circuit 26 (when the signals are outputted to the synthesis circuit 201, WLI-R and NBI-R are simultaneously outputted, WLI-G and NBI-G are outputted at a next time, and then WLI-B and NBI-B are outputted at a subsequent time, which is repeatedly performed, and when the signals are outputted to the $\gamma$ correction circuit 26, for example, in a mode for displaying the normal light observation image, WLI-R → WLI-G → WLI-B is repeated.).

The selector 102 includes the memories (not shown) in which WLI-R, WLI-G, WLI-B, NBI-R, NBI-G, and NBI-B inputted from the respective band signal conversion section 101 are stored based on the control signal from the control circuit 200 only in the mode for simultaneously displaying the normal light observation image and the narrow-band light image.

In the above explanation, the synthesis circuit 201 reduces and synthesizes the two image signals so as to laterally place the image signals. The synthesis circuit 201 may synthesize the image signals by detecting only subject image signals in the image signals (image signal portions based on a subject image, the image signals corresponding to the normal light observation image other than a margin in FIG. 6) and laterally placing only the subject image signals having been detected from the two image signals.

In the present embodiment, as shown in Fig. 9, the $\gamma$ correction circuit 26 uses different $\gamma$ correction characteristics between WL-R, WLI-G and WLI-B and NBI-R, NBI-G and NBI-B which are the frame sequential signals outputted from the selector 102. In other words, gamma-1 characteristics of Fig. 9 are used for the frame sequential color signals of WLI-R, WLI-G, and WLI-B which compose the normal light observation image, and gamma-2 characteristics of Fig. 9 are used for NBI-R, NBI-G, and NBI-B which compose the narrow-band light image, in order to achieve a high contrast.

In other words, in the case of the mode for displaying only one of the normal light observation image and the narrow-band light observation image, the $\gamma$ correction circuit 26 is fed with the control signal (the display mode for displaying only one of the normal light observation image and the narrow-band light observation image has been identified) from the control circuit 200.

As shown in Fig. 9, in the mode for displaying the normal light observation image, the $\gamma$ correction circuit 26 makes a $\gamma$ correction according to the gamma-1 characteristics based on the control signal. In a mode for displaying the narrow-band light observation image, the $\gamma$ correction circuit 26 makes a $\gamma$ correction according to the gamma-2 characteristics (In this case, the $\gamma$ correction circuit 26 does not identify the image signal based on the control signal which will be described later).

On the other hand, in the mode for simultaneously displaying the normal light observation image and the narrow-band light observation image, the $\gamma$ correction circuit 26 is fed with a sync signal outputted from the synthesis circuit 201 and is fed with the control signal (the simultaneous display mode has been identified) from the control circuit 200.

The $\gamma$ correction circuit 26 identifies, as shown in Fig. 9, the WLI image signal and the NBI image signal based on the control signal and uses the gamma-1 characteristics for the WLI image signal and the gamma-2 characteristics for the NBI image signal. For the identification of the image signals, image region information is used. For example, in the display of Fig. 8, the image signal corresponding to a left half of the screen is identified as the WLI image signal and the gamma-1 characteristics are used. The image signal corresponding to a right half is identified as the NBI image signal and the gamma-2 characteristics are used.

As described above, in the present embodiment, the respective band signal conversion section 101 generates WLI-R, WLI-G, and WLI-B for generating the normal light observation image and NBI-R, NBI-G, and NBI-B for generating the narrow-band light image, based on the RGB signals obtained by irradiation with frame sequential light of a set of the rotary filter 14. In other words, by irradiation with frame sequential light through the rotary filter 14 made up of the set of the R filter portion 14r, the G filter portion 14g, and the B filter portion 14b, the normal light observation image and the narrow-band light image can be generated in real time. Thus it is possible to simplify the configuration of the apparatus and simultaneously observe the normal light observation image and the narrow-band light image.

The synthesis circuit 201 synthesizes the normal light observation image and the narrow-band light image, so that the normal light observation image and the narrow-band light image can be simultaneously observed.

(Second Embodiment)

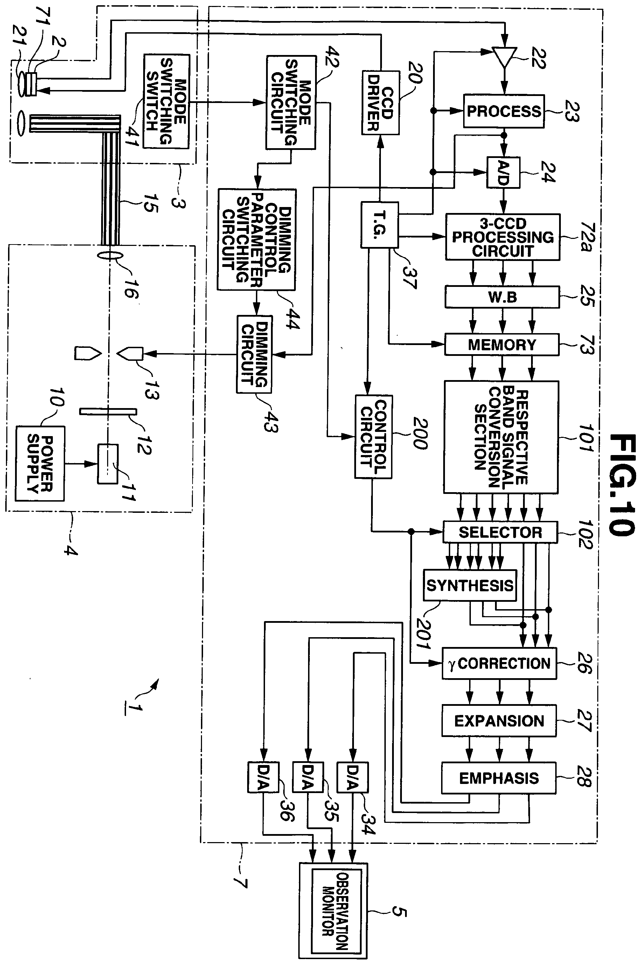

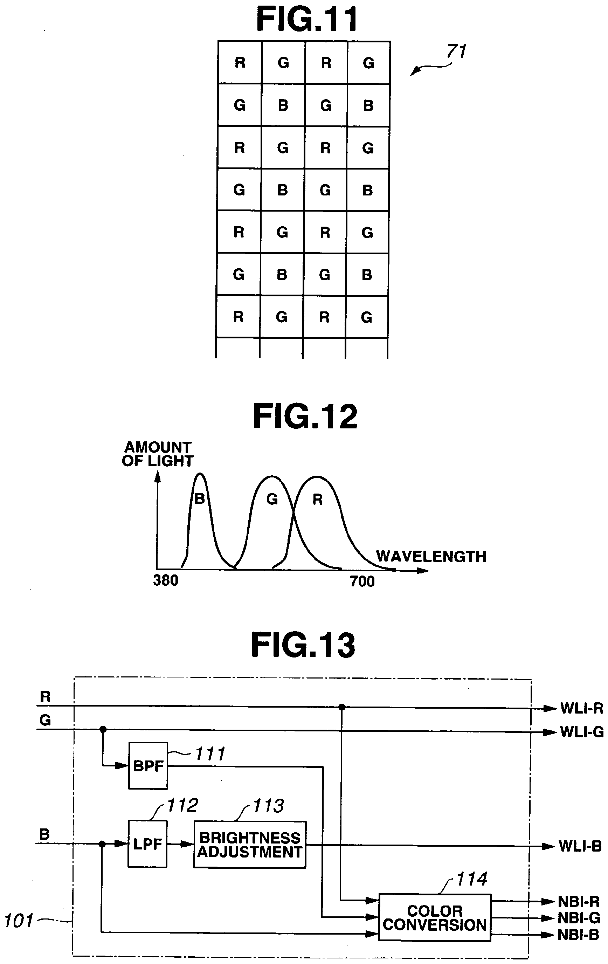

Figs. 10 to 13 show a second embodiment of the present invention. Fig. 10 is a structural diagram showing a configuration of an endoscope apparatus. Fig. 11 is a structural diagram showing a configuration of a primary color filter shown in Fig. 10. Fig. 12 shows a transmission property of the primary color filter shown in Fig. 11. Fig. 13 is a structural diagram showing a configuration of a respective band signal conversion section shown in Fig. 10.

The second embodiment is substantially the same as the first embodiment and thus only different points will be described below. The same configurations as in the first embodiment will be indicated by the same reference numerals and the explanation thereof is omitted.

In the first embodiment, the normal light observation image and the narrow-band light image are generated by frame sequential image pickup observation through the rotary filter 14. In the present embodiment, as shown in Fig. 10, white light is applied to a tissue in a body cavity and is separated into colors through a primary color filter 71, and a normal light observation image and a narrow-band light image are generated through simultaneous-type image pickup observation in which an image is picked up by a CCD 2. Fig. 11 shows the configuration of the primary color filter 71, and Fig. 12 shows the transmission property of each color filter.

In a video processor 7 of the present embodiment, as shown in Fig. 10, an RGB image signal, which is a single-CCD (single color/pixel) image signal from an A/D converter 24, is subjected to 3-CCD processing (three RGB colors/pixel) into an R signal, a G signal, and a B signal in a 3-CCD processing circuit 72a. Further, the R signal, the G signal, and the B signal which have been subjected to 3-CCD processing in the 3-CCD processing circuit 72a are subjected to white balance processing by a W.B 25 as in the first embodiment. After that, the R signal, the G signal, and the B signal which have been subjected to white balance processing are temporarily stored in a memory 73, and then the R signal, the G signal, and the B signal are read from the memory 73 and are outputted to a respective signal conversion section 101.

The respective signal conversion section 101 is configured substantially as in the first embodiment. As shown in Fig. 13, in the respective band signal conversion section 101 of the present embodiment, the R signal picked up through the primary color filter 71 is a wide-band R image signal suitable for a normal observation (see Fig. 12), and the R signal is outputted to a selector 102 as WLI-R through the respective band signal conversion section 101 and is outputted to a color conversion circuit 114. Further, the G signal picked up through the primary color filter 71 is a wide-band G image signal suitable for a normal observation (see Fig. 12), and the G signal is outputted to the selector 102 as WLI-G through the respective band signal conversion section 101 and is outputted to the color conversion circuit 114 through a BPF 111. Moreover, the B signal picked up through the primary color filter 71 is a narrow-band B image signal suitable for a narrow-band light observation (see Fig. 12). The B signal is outputted to the color conversion circuit 114 and a brightness is adjusted by a brightness adjustment circuit 113 through a LPF 112, and the B signal is outputted to the selector 102 as WLI-B.

The color conversion circuit 114 performs predetermined color conversion on the inputted image signals and outputs the signals to the selector 102 as NBI-R, NBI-G, and NBI-B.

After that, the selector 102 outputs WLI-R, WLI-G, WLI-B, and NBI-R, NBI-G, and NBI-B to a $\gamma$ correction circuit 26 or a synthesis circuit 201 based on a control signal from a control circuit 200. The synthesis circuit 201 synthesizes the inputted image signals.

In other words, in the present embodiment, the selector 102 is switched to input the six image signals (WLI-R, WLI-G, WLI-B, NBI-R, NBI-G, and NBI-B) to the synthesis circuit 201 from memories (not shown) included in the selector 102, in a display mode for simultaneously displaying the normal light observation image and the narrow-band light observation image on an observation monitor 5.

The synthesis circuit 201 reduces the two image signals of the same color (WLI-R and NBI-R, WLI-G and NBI-G, and WLI-B and NBI-B) and then synthesizes the image signals, so that a synthetic image signal (RGB image signal) is generated. The synthetic image signal is outputted to the γ correction circuit 26.

In a mode for displaying only one of the normal light observation image and the narrow-band light observation image, the selector 102 is not switched to output the image signals to the synthesis circuit 201 based on the control signal from the control circuit 200 but is switched to output the R signal, the G signal, and B signal of the normal light observation image or the narrow-band light observation image to the γ correction circuit 26.

The control circuit 200 identifies the mode based on a mode switching signal from a mode switching circuit 42 and switches the selector 102. After that, the control circuit 200 controls the R, G, and B signals in the selector 102 based on a timing signal from a T.G 37 such that the signals are outputted to the synthesis circuit 201 or the γ correction circuit 26 (when the signals are outputted to the synthesis circuit 201, WLI-R, WLI-G, WLI-B, NBI-R, NBI-G, and NBI-B are simultaneously outputted, and when the signals are outputted to the γ correction circuit 26, for example, in a mode for displaying the normal light observation image, WLI-R, WLI-G, and WLI-B are controlled to be simultaneously outputted from the selector 102).

In the above explanation, the synthesis circuit 201 reduces and synthesizes the two image signals of the same color signal so as to laterally place the image signals. The synthesis circuit 201 may synthesize the image signals by detecting only subject image signals in the image signals (image signal portions based on a subject image, the image signals corresponding to the normal light observation image other than a margin in FIG. 8) and laterally placing only the subject image signals having been detected from the two image signals.

The γ correction circuit 26 identifies, as in the first embodiment, the WLI image signal and the NBI image signal based on the control signal and uses gamma-1 characteristics for the WLI image signal and gamma-2 characteristics for the NBI image signal. For the identification of the image signals, image region information is used. For example, in display of Fig. 8, the image signal corresponding to a left half of a screen is identified as the WLI image signal and the gamma-1 characteristics are used. The image signal corresponding to a right half is identified as the NBI image signal and the gamma-2 characteristics are used.

In the case of the mode for displaying only one of the normal light observation image and the narrow-band light observation image, the γ correction circuit 26 makes a γ correction to the normal light observation image according to the gamma-1 characteristics based on the control signal from the control circuit (200), and makes a γ correction to the narrow-band light observation image according to the gamma-2 characteristics (in this case, the γ correction circuit 26 does not identify the image signal based on the control signal).

The video processor 7 of the present embodiment includes, as in the first embodiment, the γ correction circuit 26 for making a γ correction to the image signals having passed through the selector 102, an expansion circuit 27 for expanding the image signals having been subjected to the γ correction, and an emphasis circuit 28 for performing edge enhancement on the expanded image signals. The image signals from the emphasis circuit 28 are converted to analog video signals by D/A circuits 34, 35, and 36 and are outputted to the observation monitor 5.

In the present embodiment, as shown in Fig. 10, the control circuit 200 is provided. The control circuit 200 is fed with a CCD driving signal from a CCD driver 20. The control circuit 200 detects image pickup of one frame based on the CCD driving signal from the CCD driver 20, controls the selector 102, and outputs WLI-R, WLI-G, WLI-B, NBI-R, NBI-G, and NBI-B from the selector 102 to the γ correction circuit 26 or the synthesis circuit 20.

Thus the present embodiment can achieve the same effect as in the first embodiment.

(Third Embodiment)

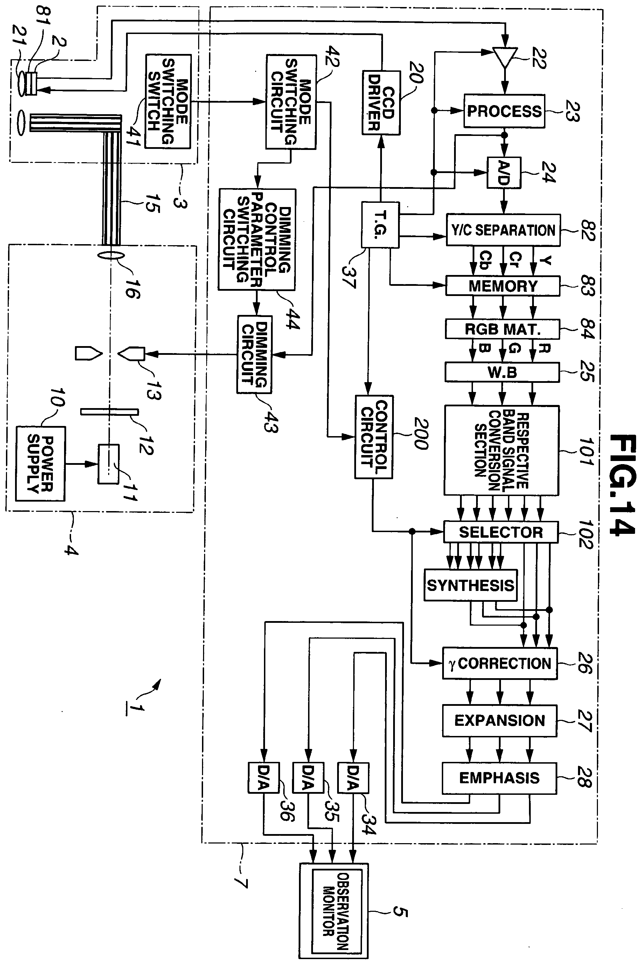

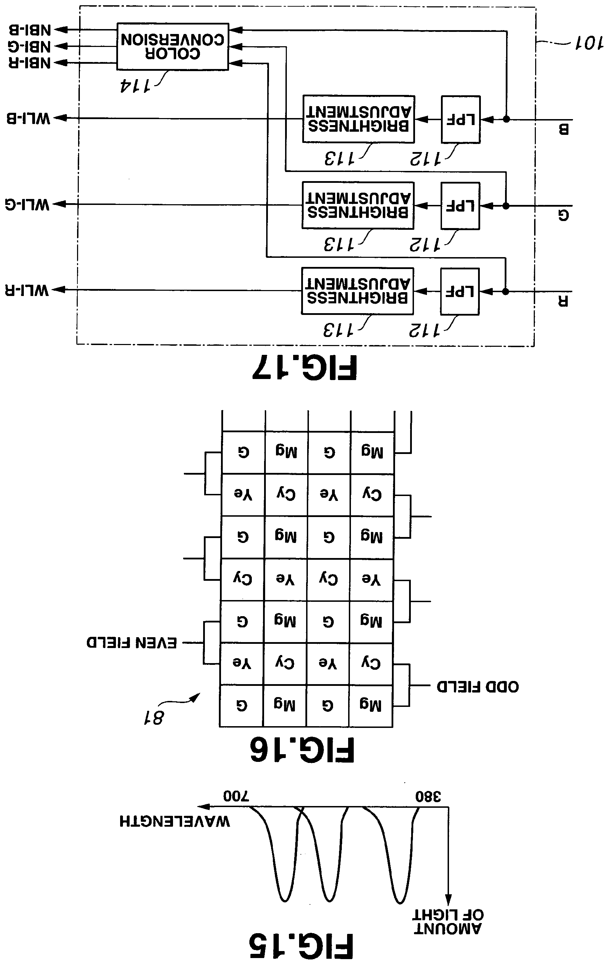

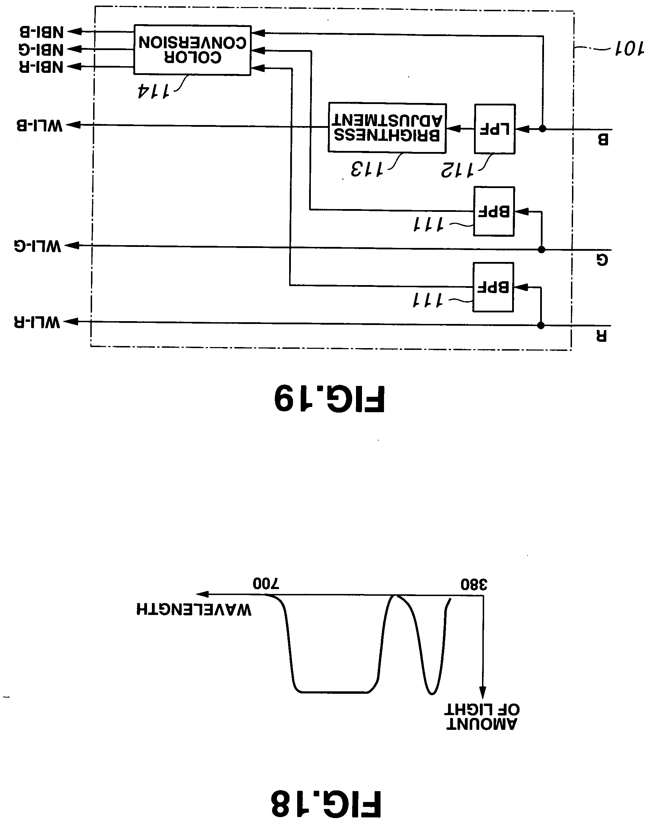

Figs. 14 to 19 show a third embodiment of the present invention. Fig. 14 is a structural diagram showing a configuration of an endoscope apparatus. Fig. 15 shows a transmission property of a heat ray cut-off filter shown in Fig. 14. Fig. 16 is a structural diagram showing a configuration of a complementary color filter shown in Fig. 14. Fig. 17 is a structural diagram showing a configuration of a respective band signal conversion section shown in Fig. 14. Fig. 18 shows a modification of a transmission property of the heat ray cut-off filter shown in Fig. 14. Fig. 19 is a structural diagram showing a configuration of a modification of a respective band signal conversion section shown in Fig. 14.

The third embodiment is substantially the same as the second embodiment and thus only different points will be described below. The same configurations as in the second embodiment will be indicated by the same reference numerals and the explanation thereof is omitted.

In the present embodiment, as shown in Fig. 14, a light source device 4 is substantially the same as in the second embodiment and a heat ray cut-off filter 12 has the transmission property of Fig. 15. Further, a complementary color filter 81 configured as shown in Fig. 16 is provided on an image pickup surface of a CCD 2, instead of a primary color filter 71.

In a video processor 7 of the present embodiment, as shown in Fig. 14, an image signal from an A/D converter 24 is subjected to Y/C separation (separated into luminance/color difference signals) in a Y/C separation circuit 82. A luminance signal Y and color difference signals Cr and Cb which have been subjected to Y/C separation are temporarily stored in a memory 83, and then the luminance signal Y and the color difference signals Cr and Cb are read from the memory 83 and are converted to RGB signals in an RGB matrix circuit 84. The R signal, the G signal, and the B signal from the RGB matrix circuit 84 are subjected to white balance processing by a W.B 25 as in the first embodiment. After that, the R signal, the G signal, and the B signal which have been subjected to white balance processing are outputted to a respective band signal conversion section 101. Configurations following the respective band signal conversion section 101 are similar to the configurations of the second embodiment.

The transmission property of the heat ray cut-off filter 12 serving as band limiting means is narrow-band characteristics as shown in Fig. 15. Thus as shown in Fig. 17, the respective band signal conversion section 101 of the present embodiment performs predetermined color conversion on the R signal, the G signal, and the B signal in a color conversion circuit 114, and then outputs the signals to a selector 102 as NBI-R, NBI-G, and NBI-B. Further, the respective band signal conversion section 101 adjusts a brightness for each of the R signal, the G signal, and the B signal in brightness adjustment circuits 113 through LPFs 112 and outputs the signals to the selector 102 as WLI-R, WLI-G, and WLI-B.

Thus the present embodiment can achieve the same effect as in the second embodiment.

The transmission property of the heat ray cut-off filter 12 is not limited to the property of Fig. 15 and the heat ray cut-off filter 12 may have a transmission property of Fig. 18. In this case, as shown in Fig. 19, the R signal and the G signal are outputted as WLI-R and WLI-G to the selector 102 through the respective band signal conversion section 101 of the present embodiment. The B signal is subjected to brightness adjustment in the brightness adjustment circuit 113 through the LPF 112 and is outputted to the selector 102 as WLI-B. Further, the R signal and the G signal are outputted to the color conversion circuit 114 through BPFs 111, are subjected to the predetermined color conversion with the B signal in the color conversion circuit 114, and then are outputted to the selector 102 as NBI-R, NBI-G, and NBI-B.

Appendix:

Appended claim 1)

In claim 1, the biological image information converting means has image signal converting means for performing image signal conversion differently between the first biological image information and the second biological image information.

Appended claim 2)

In appended claim 1, the image signal converting means is made up of contrast changing means (for example, the $\gamma$ correction circuit 26) for changing a contrast of an image signal and color converting means (for example, the color conversion circuit 114) for converting a color of the image signal.

The present invention is not limited to the foregoing embodiments and various changes and modifications can be made without changing the subject matter of the present invention.

The present application is filed claiming priority based on Japanese Patent Application No. 2006-223576 which was filed on August 18,2006. The disclosed contents are cited to a specification and claims of the present application.

**Claims**

1. An endoscope apparatus (1), comprising:

    a light source (4) adapted for generating illumination light to be applied to a subject;
    biological image information acquiring means (2) adapted for receiving a subject image of the subject having been irradiated with the illumination light from the light source (4) comprising biological image information of the subject; and
    band limiting means (14, 71) which is disposed on an optical path from the light source (4) to the biological image information acquiring means (2) and is adapted for limiting, to a predetermined bandwidth, at least one of a plurality of wavelength bands of the subject image allocated according to penetration depths of light in the subject;
    **characterized by** further comprising:

    biological image information converting means (101) adapted for generating, from the one piece of biological image information based on the subject image including band limited light limited to the predetermined bandwidth by the band limiting means (14, 71),

a first biological image information signal obtained by subjecting an image information signal corresponding to light in a wavelength band other than a wavelength band of the band limited light to a first contrast conversion, wherein

the first contrast conversion includes passing the image information signal through a band-pass filter for generating a high-contrast image signal, which corresponds to an image signal obtained by irradiation with illumination light having spectral characteristics with a narrower band than the light in the wavelength band other than the wavelength band of the band limited light and is outputted as a narrow-band image signal, and a second biological image information signal obtained by subjecting an image information signal corresponding to light in the wavelength band of the band limited light to a second contrast conversion, wherein the second contrast conversion includes passing the image information signal through a low-pass filter for generating a low-contrast image; which corresponds to an image obtained by irradiation with illumination light having spectral characteristics with a wider band than the band limited light and is outputted as a wide-band image signal; and a display image generating means (7) adapted for generating a display image to be displayed on display means (5), based on the first biological image information signal and the second biological image information signal which have been generated by the biological image information converting means (101).

2. The endoscope apparatus according to claim 1, wherein the band limiting means (14) is adapted to limit, to the predetermined bandwidth, the wavelength band of the light from the light source (4).

3. The endoscope apparatus according to claim 1, wherein the band limiting means (71) limits, to the predetermined bandwidth, the wavelength band of the subject image received by the biological image information acquiring means (2).

4. The endoscope apparatus according to claim 1 or 2, wherein the illumination light is RGB frame sequential light.

5. The endoscope apparatus according to claim 3, wherein
the illumination light is white light,
the biological image information acquiring means (2) is a CCD (2), and
the band limiting means (71) is a primary color filter (71) disposed on an image pickup surface of the CCD (2).

6. The endoscope apparatus according to claim 3, wherein the biological image information acquiring means (2) is a CCD (2) having a complementary color filter (81) disposed on an image pickup surface of the CCD (2).

7. The endoscope apparatus according to claim 1, wherein the biological image information converting means (101) has image signal converting means (26, 114) for performing image signal conversion differently between the first biological image information and the second biological image information.

8. The endoscope apparatus according to claim 7, wherein the image signal converting means (26, 114) is made up of contrast changing means (26) for changing a contrast of an image signal and/or color converting means (114) for converting a color of the image signal.

9. A signal processing method of an endoscope apparatus (1), comprising:

an illuminating step of applying illumination light generated by a light source (4) to a subject;
a biological image information acquiring step in which biological image information acquiring means (2) receives a subject image of the subject having been irradiated with the illumination light comprising biological image information of the subject;
a band limiting step of limiting, to a predetermined bandwidth, at least one of a plurality of wavelength bands of the subject image allocated according to penetration depths of light in the subject, on an optical path from the light source (4) to the biological image information acquiring means (2);
a biological image information converting step of generating, from the one piece of biological image information of the subject image including band limited light limited to the predetermined bandwidth in the band limiting step, a first biological image information signal obtained by subjecting an image information signal corresponding to light in a wavelength band other than a wavelength band of the band limited light to a first contrast conversion, wherein the first contrast conversion includes passing the image information signal through a band-pass filter for generating a high-contrast image signal, which corresponds to an image signal obtained by irradiation with illumination light having spectral characteristics with a narrower band than the light in the wavelength band other than the wavelength band of the band limited light and outputted as a narrow-band image signal, and a second biological information signal obtained by subjecting an image information signal corresponding to light in the wavelength band of the band limited light to a second contrast conversion, wherein the second contrast conversion includes passing the image information signal through a low-pass filter for generating a low-contrast image, which corresponds to an image obtained by irradiation with illumination light having spectral characteristics with a wider band than the band limited light and is outputted as a wide-band image signal; and

a display image generating step of generating a display image to be displayed on display means (5), based on the first biological image information signal and the second biological image information signal which have been generated in the biological image information converting step.

10. The signal processing method of the endoscope apparatus according to claim 9, wherein in the band limiting step, the wavelength band of the illumination light from the light source (4) is limited to the predetermined bandwidth.

11. The signal processing method of the endoscope apparatus according to claim 9, wherein in the band limiting step, the wavelength band of the subject image received by the biological image information acquiring means is limited to the predetermined bandwidth.

12. The signal processing method of the endoscope apparatus according to claim 9 or 10, wherein the illumination light is RGB frame sequential light.

13. The signal processing method of the endoscope apparatus according to claim 11, wherein
the illumination light is white light,
the biological image information acquiring step is an image pickup step performed by a CCD (2), and
the band limiting step is a band limiting step performed by a primary color filter (71) disposed on an image pickup surface of the CCD (2).

14. The signal processing method of the endoscope apparatus according to claim 11, wherein the biological image information acquiring step is an image pickup step performed by a CCD (2) having a complementary color filter (81) disposed on an image pickup surface of the CCD (2).

15. The signal processing method of the endoscope apparatus according to claim 9, wherein the biological image converting step has an image signal converting step of performing image signal conversion differently between the first biological image information and the second biological image information.

16. The signal processing method of the endoscope apparatus according to claim 15, wherein the image signal converting step is made up of a contrast changing step of changing a contrast of an image signal and/or a color converting step of converting a color of the image signal.

**Patentansprüche**

1. Endoskopgerät (1), umfassend:

eine Lichtquelle (4), die geeignet ist, um Beleuchtungslicht zu generieren, das auf ein Subjekt anzuwenden ist;
Mittel (2) zum Erfassen von biologischer Bildinformation, die geeignet sind, um ein Subjektbild des Subjekts zu empfangen, das mit dem Beleuchtungslicht von der Lichtquelle (4) bestrahlt wurde, das biologische Bildinformation des Subjekts umfasst; und
Bandbegrenzungsmittel (14, 71), die auf einem Lichtweg von der Lichtquelle (4) zu den Mitteln (2) zum Erfassen von biologischer Bildinformation angeordnet sind und geeignet sind, um mindesteins eines von einer Vielzahl von Wellenlängenbändern des Subjektbildes auf eine vorbestimmte Bandbreite zu begrenzen, die gemäß den Eindringtiefen von Licht in das Subjekt zugeteilt werden;
**dadurch gekennzeichnet, dass** es ferner umfasst:

Mittel (101) zum Konvertieren von biologischer Bildinformation, die geeignet sind, um aus der einen biologischen Bildinformation basierend auf dem Subjektbild, das bandbegrenztes Licht umfasst, das durch die Bandbegrenzungsmittel (14, 71) auf die vorbestimmte Bandbreite begrenzt ist,
ein erstes biologisches Bildinformationssignal zu generieren, das dadurch erlangt wird, dass ein Bildinformationssignal, das dem Licht in einem anderen Wellenlängenband als einem Wellenlängenband des bandbegrenzten Lichts entspricht, einer ersten Kontrastkonvertierung unterzogen wird, wobei die erste Kontrastkonvertierung das Geben des Bildinformationssignals durch ein Bandpassfilter zum Generieren eines kontrastreichen Bildsignals umfasst, das einem Bildsignal entspricht, das durch eine Bestrahlung mit Beleuchtungslicht erzielt wird, das spektrale Eigenschaften mit einem schmaleren Band als das Licht in dem anderen Wellenlängenband als dem Wellenlängenband des bandbegrenzten Lichts aufweist und als schmalbandiges Bildsignal ausgegeben wird, und

ein zweites biologisches Bildinformationssignal zu generieren, das dadurch erlangt wird, dass ein Bildinformationssignal, das dem Licht in dem Wellenlängenband des bandbegrenzten Lichts entspricht, einer zweiten Kontrastkonvertierung unterzogen wird, wobei die zweite Kontrastkonvertierung das Geben des Bildinformationssignals durch ein Tiefpassfilter zum Generieren eines kontrastarmen Bilds umfasst, das einem Bild entspricht, das durch eine Bestrahlung mit Beleuchtungslicht erzielt wird, das spektrale Eigenschaften mit einem breiteren Band als das bandbegrenzte Licht aufweist und als breitbandiges Bildsignal ausgegeben wird; und

Mittel (7) zum Generieren eines Anzeigebildes, die geeignet sind, um ein Anzeigebild zu generieren, das an Anzeigemitteln (5) anzuzeigen ist, basierend auf dem ersten biologischen Bildinformationssignal und dem zweiten biologischen Bildinformationssignal, die durch die Mittel (101) zum Konvertieren von biologischer Bildinformation generiert wurden.

2. Endoskopgerät nach Anspruch 1, wobei die Bandbegrenzungsmittel (14) geeignet sind, um das Wellenlängenband des Lichts von der Lichtquelle (4) auf die vorbestimmte Bandbreite zu begrenzen.

3. Endoskopgerät nach Anspruch 1, wobei die Bandbegrenzungsmittel (71) das Wellenlängenband des Subjektbildes, das von den Mitteln (2) zum Erfassen von biologischer Bildinformation empfangen wird, auf die vorbestimmte Bandbreite begrenzen.

4. Endoskopgerät nach Anspruch 1 oder 2, wobei das Beleuchtungslicht sequenzielles Licht von RGB-Einzelbildern ist.

5. Endoskopgerät nach Anspruch 3, wobei
das Beleuchtungslicht Weißlicht ist,
die Mittel (2) zum Erfassen von biologischer Bildinformation ein CCD (2) sind, und
die Bandbegrenzungsmittel (71) ein Primärfarbfilter (71) sind, das auf einer Bildaufnahmefläche des CCD (2) angeordnet ist.

6. Endoskopgerät nach Anspruch 3, wobei die Mittel (2) zum Erfassen von biologischer Bildinformation ein CCD (2) sind, der ein Komplementärfarbfilter (81) aufweist, das auf einer Bildaufnahmefläche des CCD (2) angeordnet ist.

7. Endoskopgerät nach Anspruch 1, wobei die Mittel (101) zum Konvertieren von biologischer Bildinformation Mittel (26, 114) zum Konvertieren von Bildsignalen umfasst, um eine Bildsignalkonvertierung zwischen der ersten biologischen Bildinformation und der zweiten biologischen Bildinformation unterschiedlich auszuführen.

8. Endoskopgerät nach Anspruch 7, wobei die Mittel (26, 114) zum Konvertieren von Bildsignalen aus Kontraständerungsmitteln (26) zum Ändern eines Kontrasts eines Bildsignals und/oder aus Farbkonvertierungsmitteln (114) zum Konvertieren einer Farbe des Bildsignals bestehen.

9. Signalverabeitungsverfahren eines Endoskopgeräts (1), umfassend:

einen Beleuchtungsschritt eines Anwendens von Beleuchtungslicht, das von einer Lichtquelle (4) generiert wird, auf ein Subjekt;
einen Schritt des Erfassens von biologischer Bildinformation, in dem die Mittel (2) zum Erfassen von biologischer Bildinformation ein Subjektbild des Subjekts empfangen, das mit dem Beleuchtungslicht bestrahlt wurde, das biologische Bildinformation des Subjekts umfasst;
einen Bandbegrenzungsschritt eines Begrenzens auf eine vorbestimmte Bandbreite mindestens eines von einer Vielzahl von Wellenlängenbändern des Subjektbildes, die gemäß den Eindringtiefen des Lichts in das Subjekt zugeteilt werden, auf einem Lichtweg von der Lichtquelle (4) zu den Mitteln (2) zum Erfassen von biologischer Bildinformation;
einen Schritt eines Konvertierens von biologischer Bildinformation, der darin besteht, aus der einen biologischen Bildinformation des Subjektbildes, das bandbegrenztes Licht umfasst, das auf die vorbestimmte Bandbreite in dem Bandbegrenzungsschritt begrenzt ist, wobei

ein erstes biologisches Bildinformationssignal dadurch erlangt wird, dass ein Bildinformationssignal, das dem Licht in einem anderen Wellenlängenband als einem Wellenlängenband des bandbegrenzten Lichts entspricht, einer ersten Kontrastkonvertierung unterzogen wird, wobei die erste Kontrastkonvertierung das Geben des Bildinformationssignals durch ein Bandpassfilter zum Generieren eines kontrastreichen Bildsignals umfasst, das einem Bildsignal entspricht, das durch eine Bestrahlung mit Beleuchtungslicht erzielt wird, das spektrale Eigenschaften mit einem schmaleren Band als das Licht in dem anderen Wellenlängenband als dem Wellenlängenband des bandbegrenzten Lichts aufweist und als schmalbandiges Bildsignal ausgegeben wird, und ein zweites biologisches Informationssignal dadurch erlangt wird, dass ein Bildinformationssignal, das Licht in dem Wellenlängenband des bandbegrenzten Lichts entspricht, einer zweiten Kontrastkonvertierung unterzogen wird, wobei die zweite Kontrastkonvertierung das Geben des Bildinformationssignals durch ein Tiefpassfilter zum Generieren eines kontrastarmen Bildes umfasst, das einem Bild entspricht, das durch eine Bestrahlung mit Beleuchtungslicht erzielt wird, das spektrale Eigenschaften mit einem breiteren Band als das bandbegrenzte Licht aufweist und als Breitbandbildsignal ausgegeben wird; und

einen Schritt eines Generieren eines Anzeigebildes, der darin besteht, ein Anzeigebild zu generieren, das an einem Anzeigemittel (5) anzuzeigen ist, basierend auf dem ersten biologischen Bildinformationssignal und dem zweiten biologischen Bildinformationssignal, die in dem Schritt des Konvertierens von biologischer Bildinformation generiert wurden.

**10.** Signalverabeitungsverfahren des Endoskopgeräts nach Anspruch 9, wobei in dem Bandbegrenzungsschritt das Wellenlängenband des Beleuchtungslichts von der Lichtquelle (4) auf die vorbestimmte Bandbreite begrenzt wird.

**11.** Signalverabeitungsverfahren des Endoskopgeräts nach Anspruch 9, wobei in dem Bandbegrenzungsschritt das Wellenlängenband des Subjektbildes, das von den Mitteln zum Erfassen von biologischer Bildinformation erfasst wird, auf die vorbestimmte Bandbreite begrenzt wird.

**12.** Signalverabeitungsverfahren des Endoskopgeräts nach Anspruch 9 oder 10, wobei das Beleuchtungslicht sequenzielles Licht von RGB-Einzelbildern ist.

**13.** Signalverabeitungsverfahren des Endoskopgeräts nach Anspruch 11, wobei

das Beleuchtungslicht Weißlicht ist,
der Schritt des Erfassens von biologischer Bildinformation ein Bildaufnahmeschritt ist, der von einem CCD (2) ausgeführt wird, und
der Bandbegrenzungsschritt ein Bandbegrenzungsschritt ist, der von einem Primärfarbfilter (71), das auf einer Bildaufnahmefläche des CCD (2) angeordnet ist, ausgeführt wird.

**14.** Signalverabeitungsverfahren des Endoskopgeräts nach Anspruch 11, wobei der Schritt des Erfassens von biologischer Bildinformation ein Bildaufnahmeschritt ist, der von einem CCD (2) ausgeführt wird, der ein Komplementärfarbfilter (81) aufweist, das auf einer Bildaufnahmefläche des CCD (2) angeordnet ist.

**15.** Signalverabeitungsverfahren des Endoskopgeräts nach Anspruch 9, wobei der Schritt des Konvertierens eines biologischen Bildes einen Bildsignalkonvertierungsschritt aufweist, der darin besteht, eine Bildsignalkonvertierung zwischen der ersten biologischen Bildinformation und der zweiten biologischen Bildinformation unterschiedlich auszuführen.

**16.** Signalverabeitungsverfahren des Endoskopgeräts nach Anspruch 15, wobei der Bildsignalkonvertierungsschritt aus einem Kontraständerungsschritt des Änderns eines Kontrastes eines Bildsignals und/oder aus einem Farbkonvertierungsschritt des Konvertierens einer Farbe des Bildsignals besteht.

**Revendications**

**1.** Dispositif endoscope (1), comprenant :

une source de lumière (4) adaptée pour générer une lumière d'éclairage devant être appliquée à un sujet ;
des moyens d'acquisition d'informations d'image biologique (2) adaptés pour recevoir une image de sujet, du sujet ayant été irradié par la lumière d'éclairage provenant de la source de lumière (4), comprenant des informations d'image biologique du sujet ; et
des moyens de limitation de bande (14, 71) qui sont disposés sur un trajet optique de la source de lumière (4) aux moyens d'acquisition d'informations d'image biologique (2) et qui sont adaptés pour limiter, à une largeur de bande prédéterminée, au moins une d'une pluralité de bandes de longueur d'onde d'une image de sujet attribuées selon des profondeurs de pénétration de la lumière dans le sujet ;

**caractérisé par le fait qu'**il comprend en outre :

des moyens de conversion d'informations d'image biologique (101) adaptés pour générer, à partir de l'information d'image biologique basée sur l'image de sujet comprenant une lumière à bande limitée, limitée à la largeur de bande prédéterminée par les moyens de limitation de bande (14, 71),

un premier signal d'informations d'image biologique obtenu en soumettant un signal d'informations d'image correspondant à une lumière dans une bande de longueur d'onde autre qu'une bande de longueur d'onde de la lumière à bande limitée, à une première conversion de contraste, la première conversion de contraste comprenant passer le signal d'informations d'image à travers un filtre passe-bande pour générer un signal d'image à contraste élevé, qui correspond à un signal d'image obtenu par irradiation avec une lumière d'éclairage ayant des caractéristiques spectrales avec une bande plus étroite que la lumière dans la bande de longueur d'onde autre que la bande de longueur d'onde de la lumière à bande limitée et est émis en tant que signal d'image à bande étroite, et

un second signal d'informations d'image biologique obtenu en soumettant un signal d'informations d'image correspondant à la lumière dans la bande de longueur d'onde de la lumière à bande limitée, à une seconde conversion de contraste, la seconde conversion de contraste comprenant faire passer le signal d'informations d'image à travers un filtre passe-bas pour générer une image à faible contraste ; qui correspond à une image obtenue par irradiation avec une lumière d'éclairage ayant des caractéristiques spectrales avec une bande plus large que la lumière à bande limitée et est émis en tant que signal d'image à bande large ; et

des moyens de génération d'image d'affichage (7) adaptés pour générer une image d'affichage à afficher sur des moyens d'affichage (5), sur la base du premier signal d'informations d'image biologique et du second signal d'informations d'image biologique qui ont été générés par les moyens de conversion d'informations d'image biologique (101).

2. Dispositif endoscope selon la revendication 1, dans lequel les moyens de limitation de bande (14) sont adaptés pour limiter, à la largeur de bande prédéterminée, la bande de longueur d'onde de la lumière provenant de la source de lumière (4).

3. Dispositif endoscope selon la revendication 1, dans lequel les moyens de limitation de bande (71) limitent, à la largeur de bande prédéterminée, la bande de longueur d'onde de l'image de sujet reçue par les moyens d'acquisition d'informations d'image biologique (2).

4. Dispositif endoscope selon la revendication 1 ou 2, dans lequel la lumière d'éclairage est une lumière séquentielle à trame RVB.

5. Dispositif endoscope selon la revendication 3, dans lequel :

la lumière d'éclairage est une lumière blanche,
les moyens d'acquisition d'informations d'image biologique (2) sont un dispositif à couplage de charge (DCC) (2), et
les moyens de limitation de bande (71) sont un filtre de couleur primaire (71) disposé sur une surface de capture d'image du DCC (2).

6. Dispositif endoscope selon la revendication 3, dans lequel les moyens d'acquisition d'informations d'image biologique (2) sont un DCC (2) ayant un filtre de couleur complémentaire (81) disposé sur une surface de capture d'image du DCC (2).

7. Dispositif endoscope selon la revendication 1, dans lequel les moyens de conversion d'informations d'image biologique (101) ont des moyens de conversion de signal d'image (26, 114) pour effectuer une conversion de signal d'image de manière différente entre la première information d'image biologique et la seconde information d'image biologique.

8. Dispositif endoscope selon la revendication 7, dans lequel les moyens de conversion de signal d'image (26, 114) sont faits de moyens de changement de contraste (26) pour changer un contraste d'un signal d'image et/ou de moyens de conversion de couleur (114) pour convertir une couleur du signal d'image.

9. Procédé de traitement de signal d'un dispositif endoscope (1), comprenant :

une étape d'éclairage consistant à appliquer à un sujet une lumière d'éclairage générée par une source de lumière (4) ;

une étape d'acquisition d'informations d'image biologique dans laquelle des moyens d'acquisition d'informations d'image biologique (2) reçoivent une image de sujet, du sujet ayant été irradié avec la lumière d'éclairage, comprenant des informations d'image biologique du sujet ;

une étape de limitation de bande consistant à limiter, à une largeur de bande prédéterminée, au moins une d'une pluralité de bandes de longueur d'onde de l'image de sujet attribuées selon des profondeurs de pénétration de la lumière dans le sujet, sur un chemin optique de la source de lumière (4) aux moyens d'acquisition d'informations d'image biologique (2) ;

une étape de conversion d'informations d'image biologique consistant à générer, à partir d'une information d'image biologique de l'image de sujet comprenant la lumière à bande limitée, limitée à la largeur de bande prédéterminée dans l'étape de limitation de bande,

un premier signal d'informations d'image biologique obtenu en soumettant un signal d'informations d'image correspondant à une lumière dans une bande de longueur d'onde autre qu'une bande de longueur d'onde de la lumière à bande limitée, à une première conversion de contraste, la première conversion de contraste comprenant faire passer le signal d'informations d'image à travers un filtre passe-bande pour générer un signal d'image à contraste élevé, qui correspond à un signal d'image obtenu par irradiation avec une lumière d'éclairage ayant des caractéristiques spectrales avec une bande plus étroite que la lumière dans la bande de longueur d'onde autre que la bande de longueur d'onde de la lumière à bande limitée et est émis en tant que signal d'image à bande étroite,

un second signal d'informations biologique obtenu en soumettant un signal d'informations d'image correspondant à une lumière dans la bande de longueur d'onde de la lumière à bande limitée, à une seconde conversion de contraste, la seconde conversion de contraste comprenant faire passer le signal d'informations d'image à travers un filtre passe-bas pour générer une image à faible contraste, qui correspond à une image obtenue par irradiation avec une lumière d'éclairage ayant des caractéristiques spectrales avec une bande plus large que la lumière à bande limitée et est émis en tant que signal d'image à bande large ; et

une étape de génération d'image d'affichage, consistant à générer une image d'affichage à afficher sur des moyens d'affichage (5), sur la base du premier signal d'informations d'image biologique et du second signal d'informations d'image biologique qui ont été générés dans l'étape de conversion d'informations d'image biologique.

10. Procédé de traitement de signal du dispositif endoscope selon la revendication 9, dans lequel, dans l'étape de limitation de bande, la bande de longueur d'onde de la lumière d'éclairage provenant de la source de lumière (4) est limitée à la largeur de bande prédéterminée.

11. Procédé de traitement de signal d'un dispositif endoscope selon la revendication 9, dans lequel, dans l'étape de limitation de bande, la bande de longueur d'onde de l'image de sujet reçue par les moyens d'acquisition d'informations d'image biologique est limitée à la largeur de bande prédéterminée.

12. Procédé de traitement de signal du dispositif endoscope selon la revendication 9 ou 10, dans lequel la lumière d'éclairage est une lumière séquentielle à trame RVB.

13. Procédé de traitement de signal du dispositif endoscope selon la revendication 11, dans lequel :

la lumière d'éclairage est une lumière blanche,

l'étape d'acquisition d'informations d'image biologique est une étape de capture d'image réalisée par un DCC (2), et

l'étape de limitation de bande est une étape de limitation de bande réalisée par un filtre de couleur primaire (71) disposé sur une surface de capture d'image du DCC (2).

14. Procédé de traitement de signal du dispositif endoscope selon la revendication 11, dans lequel l'étape d'acquisition d'informations d'image biologique est une étape de capture d'image réalisée par un DCC (2) ayant un filtre de couleur complémentaire (81) disposé sur une surface de capture d'image du DCC (2).

15. Procédé de traitement de signal du dispositif endoscope selon la revendication 9, dans lequel l'étape de conversion d'image biologique comprend une étape de conversion de signal d'image consistant à effectuer une conversion de signal d'image de manière différente entre la première information d'image biologique et la seconde information d'image biologique.

16. Procédé de traitement de signal du dispositif endoscope selon la revendication 15, dans lequel l'étape de conversion de signal d'image est faite d'une étape de changement de contraste consistant à changer un contraste d'un signal d'image et/ou d'une étape de conversion de couleur consistant à convertir une couleur du signal d'image.

FIG.1

# FIG.2

R1

14r

14

14b

G1   B1

14g

# FIG.3

AMOUNT
OF LIGHT

B   G   R

WAVELENGTH

380   700

λ11   λ12

# FIG.4

R → WLI-R

G → WLI-G

→ WLI-B

→ NBI-R

→ NBI-G

→ NBI-B

BPF — 111

112   113

B → LPF → BRIGHTNESS ADJUSTMENT

110   114   115

SYNCHRONIZATION → COLOR CONVERSION → FRAME SEQUENTIAL

101

# FIG.5

AMPLITUDE
CHARACTERISTICS

BPF

1

FREQUENCY

# FIG.6

NORMAL
LIGHT
OBSERVATION
IMAGE

5

# FIG.7

NARROW-
BAND
LIGHT
OBSERVATION
IMAGE

5

# FIG.8

NORMAL
LIGHT
OBSERVATION
IMAGE

NARROW-
BAND
LIGHT
OBSERVATION
IMAGE

5

# FIG.9

OUTPUT | γ CHARACTERISTICS

gamma-2

gamma-1

INPUT

# FIG.10

# FIG.11

| | | | |
|---|---|---|---|
| R | G | R | G |
| G | B | G | B |
| R | G | R | G |
| G | B | G | B |
| R | G | R | G |
| G | B | G | B |
| R | G | R | G |

71

# FIG.12

AMOUNT
OF LIGHT

B    G    R

WAVELENGTH

380                     700

# FIG.13

R ————————————————————————————————→ WLI-R

G ————————————————————————————————→ WLI-G

BPF 111

112    113
LPF   BRIGHTNESS
      ADJUSTMENT

B ——————————————————————————————→ WLI-B

114
COLOR
CONVERSION ————→ NBI-R
           ————→ NBI-G
101        ————→ NBI-B

**FIG.14**

# FIG.15

AMOUNT OF LIGHT

WAVELENGTH

380　　　　700

# FIG.16

| Mg | G | Mg | G |
|----|----|----|----|
| Cy | Ye | Cy | Ye |
| Mg | G | Mg | G |
| Ye | Cy | Ye | Cy |
| Mg | G | Mg | G |
| Cy | Ye | Cy | Ye |
| Mg | G | Mg | G |

ODD FIELD

EVEN FIELD

81

# FIG.17

R → LPF (112) → BRIGHTNESS ADJUSTMENT (113) → WLI-R

G → LPF (112) → BRIGHTNESS ADJUSTMENT (113) → WLI-G

B → LPF (112) → BRIGHTNESS ADJUSTMENT (113) → WLI-B

COLOR CONVERSION (114) → NBI-R, NBI-G, NBI-B

101

# FIG.18

# FIG.19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002095635 A
- JP 2006061621 A
- US 20030229270 A1
- JP 2006223576 A